# EUROPÄISCHE PATENTSCHRIFT

(11) **EP 1 931 467 B1**
(45) Veröffentlichungstag und Bekanntmachung des Hinweises auf die Patenterteilung: **13.02.2013**
(21) Anmeldenummer: 06806855.0
(22) Anmeldetag: 28.09.2006
(51) Int. Cl.: B01J 23/34, B01J 37/03, C07C 231/06, B01J 23/10, B01J 35/10

(54) **VERFAHREN ZUR HYDROLYSE VON CARBONSÄURENITRILEN IN GEGENWART EINES MANGANDIOXID-KATALYSATOR**
PROCESS FOR HYDROLYSING CARBOXYLIC ACID NITRILES IN PRESENCE OF MANGANESE DIOXIDE-CATALYST
PROCEDE POUR L'HYDROLYSE DE NITRILES D'ACIDE CARBOXYLIQUE EN PRESENCE D'UN CATALYSEUR DE DIOXYDE DE MANGANESE

(30) Priorität: 05.10.2005 DE 102005047597
(43) Veröffentlichungstag der Anmeldung: 18.06.2008
(73) Patentinhaber: Evonik Degussa GmbH, 45128 Essen (DE)
(72) Erfinder: WEIGEL, Horst, 63517 Rodenbach (DE); RONNEBURG, Axel, 63526 Erlensee (DE); WECKBECKER, Christoph, 63584 Gründau-Lieblos (DE)
(86) Internationale Anmeldenummer: PCT/EP2006/066820
(87) Internationale Veröffentlichungsnummer: WO 2007/039536

(56) Entgegenhaltungen:
- EP-A2- 0 731 079
- US-A- 4 290 923
- US-A- 5 276 185
- DATABASE WPI Week 199726 Derwent Publications Ltd., London, GB; AN 1997-285158 XP002417862 & JP 09 104665 A (DAICEL CHEM IND LTD) 22. April 1997 (1997-04-22) in der Anmeldung erwähnt

## Beschreibung

Erfindung betrifft ein katalytisches Verfahren zur Hydrolyse von Thiol- oder Thioethergruppen tragenden organischen Nitrilen zu den entsprechenden Carbonsäureamiden mit Hilfe eines Mangandioxid-Katalysators.

Dabei betrifft die Erfindung insbesondere ein katalytisches Verfahren zur Hydrolyse von 2-Hydroxy-4-methylthio-butyronitril zum 2-Hydroxy-4-methylthio-buttersäureamid, einem wertvollen Zwischenprodukt bei der Herstellung von 2-Hydroxy-4-methylthio-buttersäure, dem Hydroxyanalogen des Methionins (MHA) und dessen Salzen. Diese Stoffe finden als Futtermittelzusatz, vor allem bei der Geflügelaufzucht Verwendung. Diese, dem Methionin ähnliche Verbindungen können Methionin ersetzen und die Verwertung von Proteinen im Futter deutlich verbessern.

Bei der Hydrolyse der 2-Hydroxycarbonsäurenitrile (Cyanhydrine) handelt es sich um einen Sonderfall der Nitrilhydrolyse. Alle bekannten Verfahren zur Verseifung von Nitrilen, in denen starke Basen verwendet werden können nicht angewendet werden, weil unter diesen Reaktionsbedingungen eine Rückreaktion des Cyanhydrins zu Aldehyd und Cyanwasserstoff erfolgt.

Das 2-Hydroxy-4-methylthio-butyronitril kann auch mit hochkonzentrierten Mineralsäuren,vorzugsweise mit Schwefelsäure, in nahezu äquimolarer Menge, verseift werden. Dabei entsteht im ersten Reaktionsschritt das Amid der substituierten Buttersäure. Eine technisch gut realisierbare Trennung von 2-Hydroxy-4-methylthio-buttersäureamid und Schwefelsäure, mit dem Ziel die Schwefelsäure erneut verwenden zu können, ist jedoch nicht bekannt. Die Mineralsäure wird erst nach der Verseifung des Amids zur Hydroxycarbonsäure als Amoniumhydrogensulfat abgetrennt und in einem zusätzlichen, teuren verfahrensschritt wieder zu Schwefelsäure umgearbeitet.

Es ist auch bekannt, dass Mangandioxid die Hydrolysereaktion von Carbonsäurenitrilen zu Amiden katalysiert, wie z.B. in DE 1593320 beschrieben.

Die stöchiometrische Zusammensetzung von natürlichem sowie synthetischem Mangandioxid liegt durch den Einbau von Mangan anderer Wertigkeitsstufen in das Kristallgitter im Bereich zwischen MnO_{1,7} bis MnO_{2.0}. In den Kristallen können Fremdionen wie Kalium, Natrium enthalten sein. Mangandioxid existiert in mehreren allotropen Modifikationen. Sie unterscheiden sich stark in dem Verhalten als Katalysator. Bei Pyrolysit (beta- Mangandioxid), der stabilsten Modifikation, ist die Kristallinität am höchsten ausgeprägt. Diese Form ist katalytisch inaktiv. Die Kristallität ist in den weiteren Modifikationen weniger stark ausgeprägt und geht bis zu einem amorphen Produkt, dem Ramsdelit. Durch Röntgenbeugung können die Modifikationen zugeordnet werden. Die chemisch und katalytisch aktiven Formen des Mangandioxids sind teilweise hydratisiert und enthalten zusätzlich Hydroxylgruppen.

In zahlreichen Patentschriften werden katalytische Verfahren zur Hydrolyse von Carbonsäurenitrilen, insbesondere von 2-Hydroxynitrilen (Cyanhydrinen) mit Mangandioxid beschrieben. Diese Verfahren sind beispielsweise bestens zur Verseifung von Acetoncyanhydrin geeignet, wie US4018829 zeigt, mit dem bei der Hydrolyse von Acetoncyanhydrin zu 2-Hydroxybutyramid mit Hilfe von Mangandioxid Ausbeuten von über 90 % erreicht werden.

In diesem Zusammenhang sei auch auf die US 5 276 185 verwiesen. Dieses Dokument beschreibt einen Prozess zur Herstellung einer Amidverbindung durch Reaktion einer Nitrilverbindung mit Wasser in einer Flüssigphase in Gegenwart eines Katalysators im Wesentlichen bestehend aus Mangandioxid enthaltend ein Element aus den Gruppen III A, IV A, V A, III B, IV B, V B, VI B und VIII des Periodensystems.

Die katalytisch aktiven Modifikationen des Mangandioxids sind jedoch auch als Oxidationsmittel aktiv, was ihren Einsatz bei der Hydrolyse von Thioether- oder Thiolsubstituierten Nitrilen bedingt durch deren leichte Oxidierbarkeit deutlich einschränkt. Dabei wird vierwertiges Mangan teilweise zu dreiwertigem Mangan reduziert und der Schwefel entsprechend oxidiert.

In DE 1593320 wird ein Verfahren zur Hydrolyse von Nitrilen zu Amiden mit Hilfe von Mangandioxid beschrieben, bei dem mit aliphatischen Nitrilen Ausbeuten bis über 90 % erzielt wurden. Mit Thiodipropionitril wurden nur 8 % Ausbeute an Amid erhalten, was deutlich macht, dass herkömmliches Mangandioxid als Katalysator in Gegenwart von leicht oxidierbaren Thioethergruppen kaum geeignet ist.

In EP 0 597 298 wird eine partielle Reduktion von Mangandioxid durch eine Vorbehandlung mit Reduktionsmitteln wie Alkohol beschrieben, um die Katalysatoreigenschaften zu verbessern, insbesondere dort die Oxamid-Bildung zurückzudrängen. Mit steigendem Anteil an dreiwertigem Manganoxid geht jedoch die Aktivität des Katalysators zurück.

Die oxydierende Wirkung des Mangandioxids ist bei der Hydrolyse von Nitrilen, die leicht oxidierbare Gruppen wie Thiol- oder Thioethergruppen tragen im Allgemeinen unerwünscht. Insbesondere ist die S-Oxidation bei der Hydrolyse von 2-Hydroxy-4-methylthio-butyronitril zum 2-Hydroxy-4-methylthio-buttersäureamid, einem wichtigen Zwischenprodukt bei der Herstellung des Futtermitteladditives 2-Hydroxy-4-methylthio-buttersäure, unerwünscht. Durch Oxidation des Schwefels wird das Sulfoxid gebildet und letztlich wird damit die Ausbeute an 2-Hydroxy-4-methylthio-buttersäureamid verringert. Dieses durch Oxidation entstandene Nebenprodukt kann nicht ohne erheblichen Aufwand abgetrennt werden und führt dann zu einem verunreinigten Endprodukt, das nicht mehr ohne weiteres als Futtermitteladditiv verwendet werden kann.

Durch die mit der Oxidation des Schwefels verbundene Reduktion des Katalysators wird außerdem dessen Standzeit verkürzt, was bei einem technischen Verfahren zu wirschaftlichen Nachteilen, wie erhöhtem Katalysatorverbrauch bzw. Regenerierungsaufwand mit entsprechenden Kosten führt.

In der Patentschrift JP 09104665 wird die Herstellung von aktivem δ-Mangandioxid beschrieben und dessen Aktivität über die Größe der Oberfläche definiert. Mit diesem Katalysator wurde auch die Verseifung des 2-Hydroxy-4-methylthio-butyronitrils mit vollständigem Umsatz beschrieben. Auf die Bildung von Sulfoxid wurde in dieser Druckschrift von Seiten der Anmelderin überhaupt nicht eingegangen. Durch Nachstellen der dort angegebenen Bedingungen wurde jedoch gefunden, dass während der Reaktion das Sulfoxid des 2-Hydroxy-4-methylthio-butyronitrils in einer Selektivität von mindestens 1,6 % bis zu mehr als 4 % entsteht (Vergleichsbeispiel, Beispiel 11), was von großem Nachteil ist. Außerdem lag der Nitril-Umsatz nur bei 96,1 %, die Amid-Ausbeute bei 79,8% bei kontinuierlicher Fahrweise.

Die gleiche Anmelderin beschreibt in der Patentschrift EP 0 731 079 ein Verfahren zur Herstellung von Carbonsäuren durch Hydrolyse von Cyanhydrin mit Hilfe von Mangandioxid und anschließender Verseifung des gebildeten Amids mit Lauge zum Salz der Carbonsäure. Durch Elektrodialyse werden danach die Carbonsäure und Natronlauge voneinander getrennt. In Beispiel 3 wird in einem Säulenreaktor mit nicht näher beschriebenem δ-Mangandioxid (amorph) bei 50 °C die Bildung von MHA-Amid durch Hydrolyse des 2-Hydroxy-4-methylthio-butyronitrils mit einem Nitril-Umsatz von 100 % bei einer MHA-Amid- Ausbeute von auch 100% angegeben Dieses Ergebnis konnte genausowenig bestätigt werden. Es wurde vielmehr festgestellt, dass insbesondere in einem Säulenreaktor mit hoher Katalysatorkonzentration die oxidierende Wirkung von aktivem Mangandioxid zum Tragen kommt. Das führt bei gleichzeitiger Reduktion von Mangan⁴⁺ zu katalytisch inaktivem Mn³⁺ und vermehrt zur Bildung des Sulfoxids. Auch das Nacharbeiten dieses im Vergleich zu JP 09104665 ganz ähnlichen Beispiels zeigte, dass das Sulfoxid des 2-Hydroxy-4-methylthio-buttersäureamids mit einer Selektivität von ca. 2 % bis mehr als 4 % gebildet wird.

In der Patentschrift FR 2 750 987 wird das Problem der Oxidation des Schwefels durch Belegen von Siliziumdioxid mit Mangandioxid gelöst. Der Katalysator enthält jedoch nur 5 bis 10% wirksamen Katalysatorbestandteile. Das muss durch die Verwendung einer großen Katalysatormenge oder durch Reaktionszeiten von 17 bis 45 Stunden ausgeglichen werden. Für einen technischen Prozess ist diese Vorgehensweise unvorteilhaft.

Vor dem Hintergrund der Nachteile des Standes der Technik war die Aufgabe, die sich die Erfinder gestellt hatten, ein technisch ohne weiteres durchführbares Hydrolyseverfahren für Carbonsäurenitrile bereitzustellen, das auf

Carbonsäurenitrilen, die leicht oxidierbare Gruppen wie Thiol- oder Thioethergruppen tragen, insbesondere von entsprechenden Hydroxynitrilen anwendbar ist, mit der Maßgabe , dass die Nachteile der bekannten Verfahren, insbesondere die leichte Oxidation des Schwefels nicht oder nur in verringertem Maße stattfinden.

Gelöst werden diese sowie weitere nicht explizit genannten Aufgaben, die jedoch aus den hierin diskutierten Zusammenhängen ohne weiteres ableitbar oder erschließbar sind durch ein

Überraschenderweise wurde festgestellt, dass durch die Aufnahme von geringen Mengen an Lanthaniden in das Mangandioxid dessen unerwünschte oxidierende Wirkung gegenüber Thioether oder Thiolgruppen stark vermindert wird, ohne die katalytische Wirkung bei der Hydrolyse von Carbonsäurenitrilen nachteilig zu beeinflussen.

Verfahren zur katalytische Hydrolyse von S-R- oder S-H- substituierten Carbonsäurenitrilen der allgemeinen Formel R¹-CR²R³-CN zu den entsprechenden Carbonsäureamiden, wobei R¹, R² und R³ gleich oder verschieden sein können und Wasserstoff, mindestens einen S-R- oder S-H-substituierten Kohlenwasserstoffrest ausgewählt aus der Gruppe bestehend aus einem linearen oder ggf. verzweigten C₁-bis C₁₀- Alkylrest, einem C₆- bis C₁₀- Aryl-, einem O-, N- und/oder S-haltigen C₄-bis C₈-Heteroaryl- oder einem C₇- bis C₁₂- Aralkyl- Rest bedeuten und R³ ggf. ein Hydroxylrest ist und der Rest R einen linearen oder ggf. verzweigten C₁- bis C₄- Alkyl, C₆- bis C₁₀-Aryl-, einen O-, N- und/oder S-haltigen C₄- bis C8-Heteroaryl- oder einen C₇- bis C₁₂- Aralkyl- Rest bedeutet, und wobei die Hydrolyse mit Hilfe eines Mangandioxid-Katalysators durchgeführt wird,der mindestens 52 Gew.% Mangan und zusätzlich mindestens eine Lanthanidenverbindung sowie Lithium, Natrium, Kalium, Rubidium oder Cäsium enthält und über eine spezifische Oberfläche gemäß BET von 50 bis 550 m2/g verfügt der allgemeinen Formel:

MnMeₓM_{y}O_{z},

wobei x eine Zahl zwischen 0,05 und 0,002, y eine Zahl zwischen 0,06 und 0,02 und z eine Zahl zwischen 1,7 und 2,0 ist, Me für mindestens ein Element der Lanthaniden steht, M für ein Alkalimetell aus der Gruppe Lithium, Natrium, Kalium, Rubidium, Cäsium steht und ggf. zusätzliches Hydratwasser enthalten ist.

Erfindungsgemäß besonders geeignete Elemente aus der Gruppe der Lanthaniden sind Cer und Lanthan. Besonders bevorzugt sind daher Katalysatoren, bei denen Me für Cer und/oder Lanthan steht.

Die erfindungsgemäß verwendeten modifizierten Braunsteine enthalten neben dem Lanthanid als Fremdionen bevorzugt Lithium, Natrium oder Kalium, ganz besonders bevorzugt Kalium, das für die Wirkung bei der Katalyse der Nitrilverseifung vorteilhaft ist. Besonders bevorzugt sind somit auch Katalysatoren, bei denen M für Kalium steht.

Die erfindungsgemäß verwendeten modifizierten Braunsteine verfügen über eine spezifische Oberfläche (BET) von 50 bis 550 m²/g, vorzugsweise von 150 bis 400 m²/g, ganz besonders bevorzugt von 200 bis 300 m²/g, welche nach der Prüfvorschrift DIN66131 ermittelt wird.

Die Herstellung des Katalysators ist einfach und kann beispielsweise durch eine Behandlung von käuflichem aktiven Mangandioxid mit einer wässriger Lösung eines Salzes der Lanthaniden erfolgen. Das Verfahren zur Herstellung des Katalysators ist dadurch gekennzeichnet, dass man alkalimetallhaltiges Mangandioxid mit mindestens einem Lanthanidensalz in wäßriger Lösung oder Suspension umsetzt, den anfallenden Feststoff abtrennt, ggf. wäscht und trocknet.

Das Verfahren zur Herstellung des Katalysators wird vorzugsweise so durchgeführt, dass man alkalimetallhaltiges Mangandioxid mit mindestens einem Lanthanidensalz in wäßriger Lösung oder Suspension in den geeigneten molaren Mengenverhältnissen umsetzt, so dass der anfallende Feststoff mit der gewünschten Zusammensetzung MnMeₓM_{y}O_{z} erhalten wird, man diesen abtrennt, ggf. wäscht und anschließend trocknet.

Aktives Mangandioxid wird üblicherweise durch die Umsetzung von Kaliumpermanganat mit schwefelsaurer Lösung von Mangan-II-sulfat hergestellt, wie z.B. in EP412310 in Beispiel 1 angegeben. Das Salz der Lanthaniden kann aber auch schon bei dieser Umsetzung der Reaktionslösung zugesetzt werden.

Ein bevorzugtes Verfahren zur Herstellung des Katalysators, zeichnet sich folglich dadurch aus, dass man vor und/oder während der Umsetzung von Mangandioxid mit Lanthanidensalz das Alkalimetallhaltige Mangandioxid aus Alkalimetallpermanganat, vorzugsweise Kaliumpermanganat, und Mangan(II)sulfat in schwefelsaurer Lösung direkt herstellt. Dies ist besonders kostengünstig und man erhält dabei besonders aktive Katalysatoren.

Alkalimetallhaltiges Mangandioxid wird dabei vorzugsweise aus ca. 2 Moläquivalenten Alkalimetallpermanganat und ca.3 Moläquivalenten Mangan(II)sulfat hergestellt.

Zur Katalysatorherstellung können dabei sowohl anorganische als auch organische Lanthanidensalze eingesetzt werden, was die Katalysatorherstellung weiter vereinfacht.

Bei den anorganischen Lanthanidensalzen werden die Halogenide, Nitrate, Sulfate oder Phosphate bevorzugt eingesetzt, wobei die Phosphate am besten geeignet sind.

Bei den organischen Lanthanidensalzen werden die Carbonsäuresalze, insbesondere die Formiate oder Acetate bevorzugt eingesetzt.

Da die Katalysatoren bevorzugt Cer- und /oder Lanthan enthalten, werden zu ihrer Herstellung vorzugsweise die entsprechenden Cer- und /oder Lanthansalze eingesetzt.

Mit dreiwertigen Salzen des Cers wurde die oxidierende Wirkung am meisten verringert, diese werden daher bevorzugt verwendet. Es können aber auch vierwertige Salze des Cers zur Katalysatorpräparation eingesetzt werden.

Vorzugsweise wird daher ein Verfahren angewendet, bei dem dreiwertige und / oder vierwertige Salze des Cers zur Katalysatorpräparation eingesetzt werden.

Es hat sich auch herausgestellt, dass die aktivsten Katalysatoren erhalten werden, wenn das eingesetzte Mangandioxid in der Kristallmodifikation des α-Mangandioxids vorliegt.

Die Katalysatoren zeichnen sich dabei auch dadurch aus, dass neben der reduzierten Sulfoxidbildung bei der Hydrolyse von Thioether-haltigen Nitrilen auch gleichzeitig eine weitere Aktivierung des eingesetzten Mangandioxidkatalysators gegenüber den ohne Lanthaniden-Salz hergestellten Mangandioxid-Katalysatoren erzielt wird.

Dies macht insbesondere der Vergleich der Hydrolyseergebnisse von Beispiel 5 (Mangandioxidkatalysator ohne Lanthaniden), Beispiel 6 (Cer-haltiger Mangandioxidktalysator) und Beispiel 9 (Lanthan-haltiger Mangandioxidktalysator) deutlich. Während bei annähernd gleicher Verweilzeit der Umsatz von 95,3% (Beispiel 5) auf 99,2% (Beispiel 6) bzw. 99, 5 % (Beispiel 9) steigt, sinkt die Selektivität hinsichtlich unerwünschtem Sulfoxid in derselben Reihe von 5,2% auf 1,1% bzw. 1,9%.

Die beste Aktivierung des Katalysators und Verringerung der Sulfoxidbildung in der Hydrolysereaktion erreicht man dadurch, dass man den Katalysator nach dem oben beschriebenen erfindungsgemäßen Verfahren in wäßriger Lösung oder Suspension herstellt. Ein Mischen und Verreiben des Mangandioxids mit den Lanthanidensalzen im mehr oder weniger trockenen Zustand führt nur zu einem begrenzten Erfolg, wie der Vergleich der Beispiele 5, 6 und 7 deutlich macht.

Dadurch, dass man, ein Verfahren zur katalytischen Hydrolyse von Thioether(S-R)- oder Thiol(S-H)-, bevorzugt von S-R-, substituierten Carbonsäurenitrilen der allgemeinen Formel R¹-CR²R³-CN zu den entsprechenden Carbonsäureamiden mit Hilfe des oben beschriebenen Katalysators durchführt, wobei R¹, R² und R³ gleich oder verschieden sein können und Wasserstoff, mindestens einen von S-R oder S-H-substituierten Kohlenwasserstoffrest bedeuten und R³ gegebenenfalls ein Hydroxylrest ist, wobei der jeweilige Kohlenwasserstoffrest R¹, R², R³ einen linearen oder ggf. verzweigten C₁- bis C₁₀-Alkylrest, einen C₆- bis C₁₀- Aryl-, einen O, N und/oder S-haltigen C₄- bis C₈-Heteroaryl-Rest oder einen C₇- bis C₁₂-Aralkyl- Rest bedeutet und der Rest R einen linearen oder ggf. verzweigten C₁- bis C₄- Alkyl, C₆- bis C₁₀- Aryl-, einen O, N und/oder S-haltigen C₄-bis C₈-Heteroaryl- oder einen C₇- bis C₁₂- Aralkyl- Rest bedeutet,werden die eingangs genannten Aufgaben gelöst.

Dabei werden bevorzugt S-R- oder S-H-substituierte, insbesondere S-R- substituierte Carbonsäurenitrile eingesetzt, bei denen R¹, R², R³, R C₁- bis C₄-Alkyl, Phenyl, Naphtyl, Furyl, Thienyl, Imidazolyl, Pyridyl, Pyrimidyl oder Indolyl, Benzyl oder Naphtylmethyl bedeuten, für den Fall, dass R³ kein Hydroxylrest ist.

Das Verfahren zeichnet sich z. B. im Fall von 2-Hydroxy-4-methylthio-butyronitril durch Umsatzraten von mindestens 96 % bis zu 100% aus, je nach genauer Wahl der Bedingungen. Es können insbesondere durch ausreichend lange Reaktionszeit Nitrilumsätze von mindestens 99%, bevorzugt ≥ 99,5% und bis zu 100 % erreicht werden. Dabei werden die Selektivitäten für die unerwünschte S-Oxidationsprodukte von < 2 % der Theorie erhalten. Insbesondere bei der Hydrolyse von Thioethergruppen enthaltenden Carbonsäurenitrilen werden Selektivitäten der unerwünschten Bildung von Sulfoxidprodukten von deutlich unter 2 %d.Th, insbesondere von ≤ 1,1% d.Th. bis hin zu ≤0,2 % d.Th. ermöglicht. Dies ist von ganz besonderem Vorteil hinsichtlich der Endproduktreinheit bzw. hinsichtlich des deutlich verringerten Reinigungsaufwandes.

Bei einmaligem Gebrauch verliert der Katalysator seine Wirksamkeit nicht. Es ist daher problemlos möglich, den Katalysator, nach beendeter Hydrolysereaktion, von der Reaktionslösung zu trennen und erneut zu verwenden. Die Reaktion kann deshalb kontinuierlich und diskontinuierlich durchgeführt werden.

Überraschenderweise wurde gefunden, dass die oxidierende Wirkung des Katalysators bei wiederholtem Einsatz weiter zurückgeht, ohne dass sich die Leistung für die Hydrolysereaktion verschlechtert. Damit läßt sich die Selektivität der unerwünschten S-Oxidation bis auf <0,2% absenken. Die verwendete Katalysatormenge ist nicht kritisch und hat lediglich einen Einfluss auf die Reaktionsgeschwindigkeit.

Besonders vorteilhaft ist es, den Katalysator in einem kontinuierlichen Prozess einzusetzen. Dabei können zur Hydrolyse des Nitrils mit Katalysator befüllte Säulenreaktoren oder auch Suspensionsreaktoren verwendet werden. Auch kontinuierlich betriebene Rührkesselkaskaden oder andere dem Fachmann bekannte Ausführungsformen können gewählt werden.

Wasser ist bei dieser Hydrolysereaktion Lösungsmittel und Reaktant gleichzeitig. Die Reaktion wird vorzugsweise so ausgeführt, dass bei der Hydrolyse 10 bis 200 Mol Wasser pro Mol Nitril eingesetzt wird, besonders bevorzugt 20 bis 100 Mol Wasser. Insbesondere bei Hydrolyse von 2-Hydroxy-4-methylthio-butyronitril ist der Bereich zwischen 20 und 100 Mol Wasser pro Mol Nitril bevorzugt. Eine größere Wassermenge ist für die Umsetzung und Selektivität nicht schädlich, verringert aber die Raum-Zeit-Ausbeute.

Dort wo es für die Löslichkeit bzw. Mischbarkeit der eingesetzten Reaktanden und damit des eingesetzten Nitrils günstiger ist, aber insbesondere bei geringer Wassermenge kann als Lösungsvermittler ein inertes organisches Lösungsmittel wie C₁-bis C₄-Alkohole, C₃- bis C₆-Ketone vorzugsweise Aceton zugegeben werden.

Die Temperaturen, bei denen das erfindungsgemäße Verfahren durchgeführt wird, können in einem weiten Bereich variiert werden. Vorteilhafterweise wird die Hydrolyse des Nitrils jedoch bei Temperaturen von 10 bis 90 °C durchgeführt, dabei besonders bevorzugt wird ein Bereich von 20 bis 50 °C.

Bei Einsatz von Cyanhydrinen wird bei Anwendung höherer Temperaturen von über 90 °C die Rückbildung von Aldehyd und Cyanwasserstoff aus dem Cyanhydrin begünstigt und es werden unerwünschte Nebenprodukte gebildet. Deshalb ist hier ein Temperaturbereich von 20°C bis 50°C ganz besonders bevorzugt.

Das Verfahren wird bevorzugt zur Hydrolyse von Thioether(S-R)- oder Thiol(S-H)-substituierten Carbonsäurenitrilen der allgemeinen oben definierten Formel R¹-CR²R³-CN verwendet, bei denen R³ Hydroxyl ist.

Von den entsprechenden Cyanhydrinverbindungen R¹-CR²OH-CN werden diejenigen ganz besonders bevorzugt, bei denen für R¹, R² und R die Reste C₁- bis C₄-Alkyl als Alkylrest, Phenyl oder Naphtyl als Arylrest, Furyl, Thienyl, Imidazolyl, Pyridyl, Pyrimidyl oder Indolyl als Heteroarylrest und Benzyl oder Naphtylmethyl als Aralkyalrest stehen.

In ganz hervorragender Weise eignet sich das Verfahren zur Hydrolyse von 2-Hydroxy-4-methylthio-butyronitril zum entsprechenden 2-Hydroxy-4-methylthio-buttersäureamid.

Das Verfahren kann darüber hinaus aber auch nicht erfindungsgemäβ zur Hydrolyse nicht: schwefelhaltiger Cyanhydrine eingesetzt werden.

Hervorragende Ausbeute und selektivitäten werden dabei erreicht, wenn man Acetoncyanhydrin unter den oben angegebenen Bedingungen mit dem vorstehend beschriebenen Mangandioxid-Katalysator zum Isobuttersäureamid umsetzt, einer wichtigen Vorstufe für Methacrylsäureverbindungen. Der Vorteil hierbei ist insbesondere darin zu sehen, dass man mit dem gleichen Katalysator S-R bzw. S-H-substiuierte als auch unsubstituierte Nitrile hydrolysieren kann. Das Verfahren kann also nacheinander erfindungsgemäβ für schwefelhaltige als auch nicht erfindungsgemäβ fur nicht schwefelhaltige Nitrite in derselben Anlage ohne Katalysatorwechsel durchgeführt werden, was die flexible Einnetzbarkeit solcher Anlagen vorteilhaft erhöht.

Die folgenden Beispiele sollen das Verfahren veranschaulichen, ohne einschränkend zu wirken.

### Herstellung des Katalysators:

### Beispiel 1 :

Es wurden 30 g käufliches α-Mangandioxid des Typs HSA der Firma Erachem mit einem Gehalt von 1,6% Kalium, einem Mangangehalt von 55%, einer Korngrößeabereichs von 3,0 bis 5,5 micron und einer Oberfläche von 230 m²/g zusammen mit 811 mg Cer-III-phosphat in 300 ml VE-Wasser 24 Stunden bei 60°C gerührt. Danach wurde der Feststoff durch eine Filternutsche abgesaugt und mit 11 VE-Wasser in 3 Portionen gewaschen. Der so hergestellte Katalysator wurde 20 Stunden bei 110°C und 50 mbar getrocknet. Die spezifische Oberfläche des Katalysators betrug nach der Trocknung 239 m²/g, der Cer-Gehalt 0,97 %.

### Beispiel 2:

Beispiel 1 wurde wiederholt, jedoch statt Cer-III-phosphat 1,15 g Cer-IV-sulfat eingesetzt. Die spezifische Oberfläche des so hergestellten Katalysators betrug nach der Trocknung 266 m²/g.

### Beispiel 3:

Beispiel 1 wurde wiederholt, jedoch statt Cer-III-phosphat 1,4 g Lanthan-III-nitrat Hexahydrat eingesetzt. Die spezifische Oberfläche des so hergestellten Katalysators betrug nach der Trocknung 283 m²/g.

### Beispiel 4:

In einem Rundkolben mit Rührer und Tropftrichter wurden 14,2 g Kaliumpermanganat und 1,76 g Cer-III-phosphat zusammen mit 550 ml VE-Wasser vorgelegt und auf 85°C erwärmt. Innerhalb von 2 Minuten wurde eine Lösung aus 10,14 g Mangan-II-sulfat in 250 ml VE-Wasser mit 4,1 g konzentrierten Schwefelsäure unter kräftigem Rühren zugetropft. Die erhaltene schwarze Suspension wurde weitere 6 Stunden bei 85°C gerührt. Nach Abkühlen auf 25°C wurde der Feststoff durch Absaugen abgetrennt und mit 2 l Wasser in 5 Portionen gewaschen. Die Trocknung des Katalysators erfolgte über 14 Stunden bei 110°C und 50 mbar. Katalytische Hydrolysereaktion:

### Beispiel 5: Vergleichsbeispiel (Mangandioxid-Katalysator ohne Lanthaniden)

In einem Rundkolben mit mechanischer Rührung wurden 2,0 g unverändertes α-Mangandioxid (HSA- Typ von Erachem) und 120 g VE-Wasser in einem Wasserbad auf 40°C erwärmt und 13,1 g 2-Hydroxy-4-methylthio-butyronitril zugegeben. Nach 2 Stunden lag der Umsatz des Cyanhydrins bei 95,3 %. Die HPLC-Analyse der Reaktionslösung ergab eine Selektivität von 5,2% für das Sulfoxid des 2-Hydroxy-4-methylthiobuttersäureamids.

### Beispiel 6:

Der Versuch von Beispiel 5 wurde wiederholt, jedoch wurden als Katalysator 2,0 g des mit Cer-III-phosphat modifizierten Katalysator aus Beispiel 1 verwendet. Nach einer Reaktionszeit von 2 Stunden bei 40°C waren 99,2 % des Cyanhydrins umgesetzt. Die Selektivität für das Sulfoxid war bei 1,1 %.

### Beispiel 7 :

Es wurden 30 g käufliches α-Mangandioxid (HSA-Typ der Firma Erachem) mit einem Gehalt von 1,6% Kalium und einer Oberfläche von 230 m²/g mit 811 mg Cer-III-phosphat gemischt und in einer Reibschale fein zerrieben. Es wurden 2,0 g dieser Mischung als Katalysator bei der Hydrolyse von 13,1 g 2-Hydroxy-4-methylthio-butyronitril mit 120 g Wasser und einer Temperatur von 40°C eingesetzt. Nach 2 Stunden waren 96,8 % des Cyanhydrins umgesetzt. Die Selektivität für das unerwünschte Sulfoxid lag bei 4,2 %.

### Beispiel 8:

Der Versuch von Beispiel 5 wurde wiederholt, jedoch wurden als Katalysator 2,0 g des mit Cer-IV-sulfat modifizierten Katalysators aus Beispiel 2 verwendet. Nach einer Reaktionszeit von 2 Stunden bei 45°C waren 99,6 % des Cyanhydrins umgesetzt. Die Selektivität für das Sulfoxid lag bei 1,9 %.

### Beispiel 9:

Der Versuch von Beispiel 5 wurde wiederholt, jedoch wurden als Katalysator 2,3 g des mit Lanthannitrat modifizierten Katalysators aus Beispiel 3 verwendet. Nach einer Reaktionszeit von 2,5 Stunden bei 35°C waren 99,5 % des Cyanhydrins umgesetzt. Die Selektivität für das Sulfoxid lag bei 1,9 %.

### Beispiel 10:

In einer Versuchsreihe wurde der Katalysator recycliert. Dazu wurden in einem Rundkolben mit mechanischer Rührung 2,0 g des modifizierten Mangandioxids gemäß Beispiel 1 zusammen mit 120 g VE-Wasser in einem Wasserbad auf 40°C erwärmt und 13,1 g 2-Hydroxy-4-methylthio-butyronitril zugegeben. Nach 2,5 Stunden lag der Umsatz des Cyanhydrins bei 99,8 %. Die Selektivität für das Sulfoxid lag bei 1,1%. Danach wurde der Katalysator abfiltriert und erneut bei der Hydrolysereaktion unter den gleichen Bedingungen eingesetzt. Dieser Vorgang wurde weitere fünfmal durchgeführt. Danach lag der Cyanhydrinumsatz weiterhin bei 99,8%, die Selektivität für das Sulfoxid ging auf 0,2% zurück.

### Beispiel 11: Vergleichsbeispiel (gemäß JP 09104665, Beispiel 2)

Eine Glassäule (Durchmesser 1 cm, Länge 10 cm) wurde mit 10 g Mangandioxid-katalysator (hergestellt nach JP 09104665, Beispiel 1) gefüllt. Eine 10 Gew.% wässrige 2-Hydroxy-4-methylbutyronitril - Lösung wurde mit 10 g/h bei 40°C im Gegenstrom über die Säule gepumpt. Die ablaufende Lösung wurde mittels HPLC analysiert. Die Laufzeit betrug 100 Std. ab dem Starten der Reaktion. Dabei wurde ein Nitril-Umsatz von 96,1 % d.Th., eine Amid-Ausbeute von 79,8 % d.Th. (= 83,0 % Selektivität) und eine Ausbeute von 1,5% d.Th. MHA-Amid-Sulfoxid (= 1,6 % Selektivität) erhalten. Die in JP09104665 Beispiel 2 angegebene Amid- Ausbeute von mindestens 99,1% bei 100% Nitril-Umsatz konnte in keiner Weise bestätigt werden. Die Ausbeuten sinken bei Verwendung von genau 15 % igem wässrigem 2-Hydroxy-4-methylbutyronitril noch weiter ab, da sich dabei eine ölige, überwiegend Nitril enthaltende zweite Phase abscheidet, die den Katalysator belegt und den gleichmäßigen Umsatz verhindert.

In einem analog durchgeführten Batchansatz wurden 2 g Mangandioxidkatalysator (hergestellt nach JP 09104665, Beispiel 1) mit 0,1 Mol 2-Hydroxy-4-methylbutyronitril als 10 Gew.% wässrige Lösung bei 40°C innerhalb von 3,5 Std. umgesetzt. Die Amid-Ausbeute betrug 81,6 % d.Th. (= 84,1 % Selektivität), der Nitril-Umsatz 97,0 % d.Th. Die Ausbeute an MHA-Amid-Sulfoxid 4,0 % d.Th. (= 4,1 % Selektivität) bezogen auf eingesetztes Nitril.

## Patentansprüche

1. Verfahren zur katalytische Hydrolyse von S-R- oder S-H- substituierten Carbonsäurenitrilen der allgemeinen Formel R¹-CR²R³-CN zu den entsprechenden Carbonsäureamiden, wobei R¹, R² und R³ gleich oder verschieden sein können und Wasserstoff, mindestens einen S-R- oder S-H-substituierten Kohlenwasserstoffrest ausgewählt aus der Gruppe bestehend aus einem linearen oder ggf. verzweigten C₁-bis C₁₀- Alkylrest, einem C₆- bis C₁₀- Aryl-, einem O-, N- und/oder S-haltigen C₄-bis C₈-Heteroaryl- oder einem C₇- bis C₁₂- Aralkyl- Rest bedeuten und R³ ggf. ein Hydroxylrest ist und der Rest R einen linearen oder ggf. verzweigten C₁- bis C₄- Alkyl, C₆- bis C₁₀-Aryl-, einen O-, N- und/oder S-haltigen C₄- bis C8-Heteroaryl- oder einen C₇- bis C₁₂- Aralkyl- Rest bedeutet, und wobei die Hydrolyse mit Hilfe eines Mangandioxid-Katalysators durchgeführt wird,der mindestens 52 Gew.% Mangan und zusätzlich mindestens eine Lanthanidenverbindung sowie Lithium, Natrium, Kalium, Rubidium oder Cäsium enthält und über eine spezifische Oberfläche gemäß BET von 50 bis 550 m2/g verfügt der allgemeinen Formel:
MnMeₓM_{y}O_{z},
wobei x eine Zahl zwischen 0,05 und 0,002, y eine Zahl zwischen 0,06 und 0,02 und z eine Zahl zwischen 1,7 und 2,0 ist, Me für mindestens ein Element der Lanthaniden steht, M für ein Alkalimetell aus der Gruppe Lithium, Natrium, Kalium, Rubidium, Cäsium steht und ggf. zusätzliches Hydratwasser enthalten ist.

2. Verfahren gemäß Anspruch 1 **dadurch gekennzeichnet, dass** man S-R- oder S-H- substituierte Carbonsäurenitrile einsetzt, bei denen R¹, R², R³, R bedeuten C₁- bis C₄-Alkyl, Phenyl, Naphtyl, Furyl, Thienyl, Imidazolyl, Pyridyl, Pyrimidyl, Indolyl, Benzyl oder Naphtylmethyl unter der Voraussetzung, dass R³ kein Hydroxylrest ist.

3. Verfahren gemäß Anspruch 1, **dadurch gekennzeichnet, dass** man S-R- oder S-H- substituierte Carbonsäurenitrile einsetzt, bei denen R³ Hydroxyl bedeutet.

4. Verfahren gemäß Anspruch 3, **dadurch gekennzeichnet, dass** man S-R- oder S-H- substituierte Carbonsäurenitrile einsetzt, bei denen R¹, R², R bedeuten C₁-bis C₄-Alkyl, Phenyl, Naphtyl, Furyl, Thienyl, Imidazolyl, Pyridyl, Pyrimidyl oder Indolyl, Benzyl oder Naphtylmethyl.

5. Verfahren gemäß Anspruch 4, **dadurch gekennzeichnet, dass** 2-Hydroxy-4-methylthio-butyronitril zur Hydrolyse eingesetzt wird.

6. Verfahren gemäß mindestens einem der Ansprüche 1 bis 5, **dadurch gekennzeichnet, dass** der Katalysator, nach beendeter Reaktion, von der Reaktionslösung getrennt und erneut verwendet wird.

7. Verfahren gemäß mindestens einem der Ansprüche 1 bis 6, **dadurch gekennzeichnet, dass** der Katalysator in einem kontinuierlichen Prozess eingesetzt wird.

8. Verfahren gemäß Anspruch 7, **dadurch gekennzeichnet, dass** zur Hydrolyse des Nitrils-Säulenreaktoren oder Suspensionsreaktoren verwendet werden

9. Verfahren gemäß mindestens einem der Ansprüche 1 bis 8, **dadurch gekennzeichnet, dass** bei der Hydrolyse 10 bis 200 Mol Wasser pro Mol Nitril eingesetzt wird.

10. Verfahren gemäß mindestens einem der Ansprüche 1 bis 9, **dadurch gekennzeichnet, dass** die Hydrolyse des Nitrils in Gegenwart eines inerten organischen Lösungsmittels durchgeführt wird.

11. Verfahren gemäß mindestens einem der Ansprüche 1 bis 10, **dadurch gekennzeichnet, dass** die Hydrolyse des Nitrils bei Temperaturen von 10 bis 90 °C durchgeführt wird.

12. Verfahren nach einem der Ansprüche 1-10, **dadurch gekennzeichnet, dass** beim Mangandioxid-Katalysator Me für Cer und/oder Lanthan steht.

13. Verfahren nach einem der Ansprüche 1-10, **dadurch gekennzeichnet, dass** die spezifische Oberfläche (BET) des Mangandioxid-Katalysators 150 bis 400 m²/g beträgt.

## Claims

1. Process for catalytic hydrolysis of S-R or S-H-substituted carbonitriles of the general formula R¹-CR²R³-CN to the corresponding carboxamides, where R¹, R² and R³ may be the same or different and are each hydrogen, at least one S-R- or S-H-substituted hydrocarbon radical selected from the group consisting of a linear or optionally branched C₁- to C₁₀-alkyl radical, a C₆- to C₁₀-aryl radical, an O-, N- and/or S-containing C₄- to C₈-heteroaryl radical or a C₇- to C₁₂-aralkyl radical and R³ is optionally a hydroxyl radical and the R radical is a linear or optionally branched C₁- to C₄-alkyl, C₆- to C₁₀-aryl radical, an O-, N- and/or S-containing C₄- to C₈-heteroaryl radical or a C₇- to C₁₂-aralkyl radical, and wherein the hydrolysis is performed with the aid of a manganese dioxide catalyst which contains at least 52% by weight of manganese and additionally at least one lanthanide compound, and also lithium, sodium, potassium, rubidium or caesium, and has a specific BET surface area of 50 to 550m²/g, of the general formula:
MnMeₓM_{y}O_{z},
where x is between 0.05 and 0.002, y is between 0.06 and 0.02 and z is between 1.7 and 2.0, Me is at least one element of lanthanides, M is an alkali metal from the group comprising lithium, sodium, potassium, rubidium, caesium, and additional water of hydration may be present.

2. Process according to Claim 1,
**characterized in that**
S-R- or S-H-substituted carbonitriles are used in which R¹, R², R³, R are each C₁- to C₄-alkyl, phenyl, naphthyl, furyl, thienyl, imidazolyl, pyridyl, pyrimidyl or indolyl, benzyl or naphthylmethyl, with the prerequisite that R³ is not a hydroxyl radical.

3. Process according to Claim 1, **characterized in that** S-R- or S-H-substituted carbonitriles are used in which R³ is hydroxyl.

4. Process according to Claim 3, **characterized in that** S-R or S-H-substituted carbonitriles are used in which R¹, R², R are each C₁- to C₄-alkyl, phenyl, naphthyl, furyl, thienyl, imidazolyl, pyridyl, pyrimidyl or indolyl, benzyl or naphthylmethyl.

5. Process according to Claim 4, **characterized in that** 2-hydroxy-4-methylthiobutyronitrile is used for the hydrolysis.

6. Process according to at least one of Claims 1 to 5, **characterized in that** the catalyst, when the reaction has ended, is separated from the reaction solution and used again.

7. Process according to at least one of Claims 1 to 6, **characterized in that** the catalyst is used in a continuous process.

8. Process according to Claim 7, **characterized in that** the nitrile is hydrolysed by using column reactors or suspension reactors.

9. Process according to at least one of Claims 1 to 8, **characterized in that** 10 to 200 mol of water are used per mole of nitrile in the hydrolysis.

10. Process according to at least one of Claims 1 to 9, **characterized in that** the hydrolysis of the nitrile is performed in the presence of an inert organic solvent.

11. Process according to at least one of Claims 1 to 10, **characterized in that** the hydrolysis of the nitrile is performed at temperatures of 10 to 90°C.

12. Process according to any of Claims 1-10, **characterized in that** Me is cerium and/or lanthanum in the manganese dioxide catalyst.

13. Process according to one of Claims 1-10, **characterized in that** the specific surface area (BET) of the manganese dioxide catalyst is 150 to 400 m²/g.

## Revendications

1. Procédé pour l'hydrolyse catalytique de nitriles d'acides carboxyliques S-R- ou -S-H-substitués de formule générale R¹-CR²R³-CN en les carboxamides correspondants, R¹, R² et R³ pouvant être identiques ou différents et représentant un atome d'hydrogène, au moins un radical hydrocarboné S-R- ou -S-H-substitué, choisi dans le groupe constitué par un radical alkyle en C₁-C₁₀ linéaire ou éventuellement ramifié, un radical aryle en C₆-C₁₀, un radical hétéroaryle en C₄-C₈ contenant O, N et/ou S ou un radical aralkyle en C₇-C₁₂ et R³ étant éventuellement un radical hydroxy et le radical R représentant un radical alkyle en C₁-C₄ linéaire ou éventuellement ramifié, un radical aryle en C₆-C₁₀, un radical hétéroaryle en C₄-C₈ contenant O, N et/ou S ou un radical aralkyle en C₇-C₁₂, et l'hydrolyse étant effectuée à l'aide d'un catalyseur au dioxyde de manganèse, qui contient au moins 52 % en poids de manganèse et en outre au moins un composé de type lanthanide ainsi que du lithium, du sodium, du potassium, du rubidium ou du césium et est doté d'une surface spécifique selon BET de 50 à 550 m²/g, de formule générale :
MnMeₓM_{y}O_{z},
x étant un nombre compris entre 0,05 et 0,002, y étant un nombre compris entre 0,06 et 0,02 et z étant un nombre compris entre 1,7 et 2,0, Me représentant au moins un élément des lanthanides, M représentant un métal alcalin choisi dans le groupe constitué par le lithium, le sodium, le potassium, le rubidium, le césium et de l'eau d'hydratation supplémentaire étant éventuellement contenue.

2. Procédé selon la revendication 1, **caractérisé en ce qu'**on utilise des nitriles d'acides carboxyliques S-R- ou -S-H-substitués, dans lesquels R¹, R², R³, R représentent un groupe alkyle en C₁-C₄, phényle, naphtyle, furyle, thiényle, imidazolyle, pyridyle, pyrimidyle, indolyle, benzyle ou naphtylméthyle, étant entendu que R³ n'est pas un radical hydroxy.

3. Procédé selon la revendication 1, **caractérisé en ce qu'**on utilise des nitriles d'acides carboxyliques S-R- ou -S-H-substitués, dans lesquels R³ représente le radical hydroxy.

4. Procédé selon la revendication 3, **caractérisé en ce qu'**on utilise des nitriles d'acides carboxyliques S-R- ou -S-H-substitués, dans lesquels R¹, R², R représentent un groupe alkyle en C₁-C₄, phényle, naphtyle, furyle, thiényle, imidazolyle, pyridyle, pyrimidyle ou indolyle, benzyle ou naphtylméthyle.

5. Procédé selon la revendication 4, **caractérisé en ce que** pour l'hydrolyse on utilise le 2-hydroxy-4-méthylthio-butyronitrile.

6. Procédé selon au moins l'une quelconque des revendications 1 à 5, **caractérisé en ce qu'**une fois la réaction terminée on sépare le catalyseur de la solution réactionnelle et on l'utilise à nouveau.

7. Procédé selon au moins l'une quelconque des revendications 1 à 6, **caractérisé en ce que** le catalyseur est utilisé dans un processus continu.

8. Procédé selon la revendication 7, **caractérisé en ce que** pour l'hydrolyse du nitrile on utilise des réacteurs de type colonne ou des réacteurs à suspension.

9. Procédé selon au moins l'une quelconque des revendications 1 à 8, **caractérisé en ce que** dans l'hydrolyse on utilise de 10 à 200 moles d'eau par mole de nitrile.

10. Procédé selon au moins l'une quelconque des revendications 1 à 9, **caractérisé en ce qu'**on effectue l'hydrolyse du nitrile en présence d'un solvant organique inerte.

11. Procédé selon au moins l'une quelconque des revendications 1 à 10, **caractérisé en ce qu'**on effectue l'hydrolyse du nitrile à des températures de 10 à 90 °C.

12. Procédé selon l'une quelconque des revendications 1 à 10, caractérisé en ce dans le catalyseur au dioxyde de manganèse Me représente le cérium et/ou le lanthane.

13. Procédé selon l'une quelconque des revendications 1 à 10, caractérisé en ce la surface spécifique (BET) du catalyseur au dioxyde de manganèse vaut de 150 à 400 m²/g.
